# EUROPÄISCHE PATENTSCHRIFT

(11) **EP 0 941 465 B1**
(45) Veröffentlichungstag und Bekanntmachung des Hinweises auf die Patenterteilung: **19.03.2003**
(21) Anmeldenummer: 97951990.7
(22) Anmeldetag: 27.11.1997
(51) Int. Cl.: G01N 15/02

(54) **VERFAHREN ZUR UNTERSCHEIDUNG ODER ERFASSUNG VON PARTIKELN IN EINER PROBE DURCH IDENTIFIZIERUNG VON SIGNALABSCHNITTEN ZEITAUFGELÖSTER, OPTISCHER ROHSIGNALE AUS DER PROBE AUF DER BASIS VON EINZELPHOTONENDETEKTION**
METHOD FOR DIFFERENTIATING OR DETECTING PARTICLES IN A SAMPLE BY IDENTIFYING SIGNAL SEGMENTS OF TIME-RESOLVED, OPTICAL RAW SIGNALS FROM THE SAMPLE ON THE BASIS OF SINGLE PHOTON DETECTION
PROCEDE POUR DIFFERENCIER OU DETECTER DES PARTICULES DANS UN ECHANTILLON PAR IDENTIFICATION DE SEGMENTS DE SIGNAUX BRUTS OPTIQUES A RESOLUTION TEMPORELLE PROVENANT DE L'ECHANTILLON, SUR LA BASE DE LA DETECTION MONOPHOTONIQUE

(30) Priorität: 27.11.1996 DE 19649048
(43) Veröffentlichungstag der Anmeldung: 15.09.1999
(73) Patentinhaber: Evotec OAI AG, 22525 Hamburg (DE)
(72) Erfinder: SEIDEL, Claus, D-37085 Göttingen (DE); GÜNTHER, Rolf, D-22769 Hamburg (DE); LÜPKE, Stefan, D-22159 Hamburg (DE)
(74) Vertreter: Meyers, Hans-Wilhelm, Dr.Dipl.-Chem.
(86) Internationale Anmeldenummer: EP9706622
(87) Internationale Veröffentlichungsnummer: WO98023942

(56) Entgegenhaltungen:
- WO-A-96/27798
- US-A- 4 979 824
- KELLER R A ET AL: "SINGLE-MOLECULE FLUORESCENCE ANALYSIS IN SOLUTION" APPLIED SPECTROSCOPY, Bd. 50, Nr. 7, Juli 1996, Seiten 12A-32A, XP000642337 in der Anmeldung erwähnt

## Beschreibung

Die vorliegende Erfindung betrifft ein Verfahren zur Unterscheidung oder Erfassung von Partikeln in einer Probe durch Identifizierung von Signalabschnitten zeitaufgelöster, optischer Rohsignale aus der Probe auf der Basis von Einzelphotonendetektion gemäß Anspruch 1.

Die Methode der Fluoreszenz-Korrelationsspektroskopie (FCS) (WO 94/16313) sowie andere konfokale Fluoreszenztechniken, wie sie in der Offenlegungsschrift WO 96/13744 sowie der europäischen Offenlegungsschrift EP0836090.0 beschrieben sind, eignen sich in besonderer Weise für sogenannte homogene Assays im Submikrolitermaßstab. Die letztgenannte Anmeldung beschreibt eine Methode zur Analyse von Proben durch wiederholte Messung der Anzahl von Photonen pro definiertem Zeitintervall von Licht, welches von den Partikeln in der Probe emittiert, gestreut und/oder reflektiert wird, und Bestimmung der Verteilung der Anzahl von Photonen in den jeweiligen Zeitintervallen, dadurch gekennzeichnet, daß die Verteilung der spezifischen Helligkeiten der Partikel aus der Verteilung der Anzahl von Photonen bestimmt wird. Bei diesen Techniken beträgt das eigentliche Meßvolumenelement weniger als 10⁻¹² 1. Unter homogenen Assays versteht man Analyseverfahren, bei denen alle reagierenden Komponenten bis zur Signalerfassung in einem Meßkompartiment verbleiben. Überschußkomponenten werden im Gegensatz zu den sog. ELISA-Verfahren nicht zuvor aus dem Reaktionsgemisch abgetrennt. Die oben genannten Fluoreszenztechniken funktionieren besonders gut beim Einsatz markierter Reagenzien im Bereich zwischen 1 pM und 0,1 µM. Jedoch verlangen die kinetischen Randbedingungen für eine chemische Nachweisreaktion, die aus Praktikabilitätsgründen im Sekunden- bis Minutenbereich ablaufen soll, den Einsatz markierter Nachweisreagenzien im Überschuß über einen Analyten im nanomolaren Bereich und darüber. Dies bedeutet oft eine große experimentelle Schwierigkeit beim Durchführen homogener Assays, wenn das resultierende Reaktionsprodukt eine oder mehr als eine Größenordnung geringer konzentriert ist als das Überschußreagenz.

Bei homogenen Assays mit konfokalen Meßtechniken sind oftmals keine Möglichkeiten gegeben, die Überschußkomponente mit einfachen Mitteln aus dem Meßvolumen fernzuhalten. Nur für gegensätzlich geladene Partikel sind Verfahren beschrieben worden (WO 94/16313), um durch elektrische Felder auch innerhalb eines Reaktionsvolumens Überschußkomponenten und Reaktionsprodukt voneinander zu trennen. Für viele wirtschaftlich außerordentlich bedeutungsvolle diagnostische Nachweismethoden sind derartige Verfahren jedoch leider ungeeignet. Dies gilt insbesondere z. B. für die Analytik pharmakologisch bedeutsamer Rezeptoren, z. B. auf Vesikeln, deren Interaktion mit im Überschuß vorhandenen freien markierten Liganden analysiert werden soll. Viren, Bakterien, Zellen, Beads, Partikelfluide (z. B. mit oberflächengebundenen spezifischen Chemikalien, wie sie aus der kombinatorischen Chemie bekannt sind) oder auch nur große Molekülaggregate wie Ribosomen lassen sich naturgemäß nur in äußerst geringen Konzentrationen vermessen, so daß der Einsatz von Reagenz im Überschuß unvermeidlich ist.

Es sind bereits für homogene Assayverfahren Möglichkeiten beschrieben worden, die trotz Einsatz von markiertem Nachweisreagenz im Überschuß nur dann zu optischen Lumineszenzsignalen führen, wenn Komplexe zwischen dem Nachweisreagenz und dem nachzuweisenden Partikel gebildet wurden. Es sind dies z. B. die Verfahren des Szintillations-Nachbarschafts-Assays (SPA; scintillation proximity assay), des Energy Transfer Assays durch Bildung diverser Donor-Acceptor-Komplexe oder von Reaktionsprodukten, die eine Lumineszenzlöschung rückgängig machen.

In der konfokalen Fluorimetrie hat sich die Methode der Kreuzkorrelation bewährt. Bei der Kreuzkorrelation weist z. B. das Nachweisreagenz einen Marker A auf, während das nachzuweisende Partikel den Marker B aufweist. Es werden zwar sowohl die Signale A' des Nachweisreagenzes als auch B' des nachzuweisenden Partikels registriert, jedoch im Rahmen der Kreuzkorrelation nur diejenigen Signale verwertet, bei denen beide Signale A' und B' im identischen Zeitintervall registriert werden. Dies ist bei Vorliegen eines Komplexes aus Nachweisreagenz und nachzuweisendem Partikel der Fall. In einer weiteren Variante können auch einzelne Moleküle des Nachweisreagenzes wahlweise mit den Markern A* bzw. B* markiert sein. Diese Verfahren führen nicht mehr zu zufriedenstellenden Resultaten, wenn die Überschußkomponente in ihrer Konzentration deutlich mehr als einen Faktor 10 vom nachzuweisenden Komplex differiert.

Bei verschiedenen Signalverarbeitungsverfahren hat sich in der Vergangenheit die Aufgabe gestellt, Signale vom Hintergrundrauschen zu trennen. Für diese Aufgaben haben sich z. B. der Einsatz von spektraler Filterung, Lock-In- und Koinzidenztechniken bewährt.

Spektrale Filterung läßt sich bei optischen Signalen häufig einfach durchführen. So wird etwa bei der konfokalen Mikroskopie oder der Fluoreszenzkorrelationsspektroskopie das Fluoreszenzsignal vom Streulicht und der Ramanemission des Lösungsmittels durch Interferenz- oder Farbglasfilter getrennt. Wo es möglich ist, erfolgt die Auflösung des Signals in ein Spektrum durch ein Prisma oder ein Gitter. In vielen Fällen reicht diese Art Filterung jedoch nicht aus. Zur Aufnahme eines kompletten Spektrums wird eine größere Lichtintensität benötigt, um eine minimale Statistik über den spektralen Bereich zu bekommen. Einzelne Partikel, insbesondere einzelne Moleküle, senden dafür jedoch meist nicht ausreichend viele Photonen aus. Filter schränken zwar das zu vermessende Spektrum ein, setzen jedoch einen detektierbaren spektralen Unterschied voraus.

Die Filterung des Signals nach der elektrischen Umwandlung in Zählerpulse mit Hilfe der schnellen Fouriertransformation (FFT; Fast Fourier Transformation) setzt eine Zuordnungsmöglichkeit des Signals nach Frequenzen voraus. Entsprechend schwach ist die Filterleistung der FFT und verwandter Techniken bei der Analyse von Partikelemissionen, deren Bewegung statistisch durch Diffusion und nicht regelmäßig verläuft. Lock-In Techniken verlangen ebenfalls ein periodisches Signal als Grundsignal.

Koinzidenztechniken benötigen ein zweites Signal als Triggersignal. Ein solches Signal ist von einem Partikel oder gar einem kleinen Molekül nur schwer zu erhalten. Bei Verwendung eines gepulsten Lasers treten Streulichtphotonen in Koinzidenz mit dem Puls der Lichtquelle auf, Lumineszenzphotonen dagegen zumeist um einige Nanosekunden später. Diese Zeitdifferenz wird u. a. zur Berechnung der Fluoreszenzlebensdauer verwendet. Bei der Verwendung einer zeitlich konstanten Lichtquelle, wie es beispielsweise bei der FCS meist der Fall ist, entfällt jedoch eine solche Möglichkeit.

In Tellinghuisen et al. ("Analysis of Fluorescence Lifetime Data for Single Rhodamine Molecules in Flowing Sample Streams", Analytical Chemistry 66, No. 1, 64-72, 1994) wird ein Verfahren für die Fluoreszenz-Lebensdauerspektroskopie beschrieben, das dazu dienen soll, die von der Lichtquelle, in diesem Fall von einem Laser stammenden Photonen aus dem Gesamtsignal herauszufiltern. Dazu wird der Signalzählerstrom mit dem Triggersignal, d. h. dem Anregungspuls des gepulsten Lasers, verglichen. Kommt ein Photon im Rahmen der Lichtgeschwindigkeit zeitgleich mit dem Anregungspuls an, so wird es als Streulichtpuls identifiziert und gelöscht. Ein Nachteil dieses Verfahrens ist, daß Detektorpulse, die nicht gleichzeitig mit dem Anregungspuls erzeugt werden, nicht erkannt werden. Beispiele hierfür sind Dunkelströme des Detektors. Darüber hinaus werden Fluoreszenzphotonen, wenn sie innerhalb der Grenze der Zeitauflösung des Triggersignals mit dem Anregungspuls zusammenfallen, ebenfalls gelöscht. Dies kann für die nachfolgende Signalverarbeitung zu nicht akzeptablen Verzerrungen führen. Bei Messungen von Relaxationssignalen, wie sie die Fluoreszenz-Lebensdauer darstellt, ergibt sich der zusätzliche Nachteil, daß der stärkste Teil des Signals gelöscht wird und sich das Signal-Rausch-Verhältnis demzufolge dramatisch verschlechtert.

In Keller et al. ("Single-Molecule Fluorescence Analysis in Solution", Applied Spectroscopy 50, No. 7, 12A-32A, 1996) wird ein weiteres Verfahren für die Bestimmung der Fluoreszenzlebensdauer beschrieben. In diesem Verfahren werden die Zeitdifferenzen zwischen am Detektor eintreffenden, zeitlich aufeinanderfolgenden Impulsen bestimmt, indem die Anzahl von Triggerpulsen, die mit 100 kHz erzeugt werden, zwischen zwei aufeinanderfolgenden Photonen gezählt wird. Diese Zahlen werden in aufeinander folgenden Kanälen eines MCS (engl. "multichannel scaler") gespeichert. Anschließend wird dieses MCS-Signal einer zeitlichen, schnellen Fouriertransformation (FFT) zur Glättung unterworfen. Liegen nach der FFT wenigstens 5 Zeitdifferenzen des geglätteten Signals unterhalb eines visuell bestimmten Schwellenwertes, so wird das Signal für die gesamte Zeitdauer, während der die Zeitdifferenzen kontinuierlich unterhalb des visuell bestimmten Schwellenwertes liegen, als zusammengehörig erachtet und als Fluoreszenzanteil, im wissenschaftlichen Sprachgebrauch seit einiger Zeit "burst" genannt, gewertet. Anschließend wird das so gefilterte Signal in bezug auf die Fluoreszenzlebensdauer ausgewertet. Allerdings verwenden die Autoren hier nur Signalanteile, bei denen mehr als 25 Zeitdifferenzen unterhalb des visuell bestimmten Schwellwertes liegen.

Nachteile des beschriebenen Verfahrens liegen in der Verzögerung der Auswertung und der Beschränkung der Meßzeit. Die zur Glättung der Daten verwendete und notwendige FFT wird über ein 2ⁿ Meßpunkte umfassendes Intervall ausgewertet, wobei die Meßpunkte wegen des in der FFT verwendeten mathematischen Verfahrens in einem Speicher, in diesem Fall im MCS, präsent sein müssen. Erst nach der FFT und einer "visuellen" Entscheidungsprozedur kann eine Zuordnung der Impulse getroffen werden. Ein solches Verfahren ist bei Messungen, in denen Zählraten von weit weniger als 100 pro Millisekunde auftreten, bei geringen Konzentrationen der fluoreszierenden Partikel (« 1 Partikel pro Meßvolumenelement), noch durchführbar.

Neben der Bestimmung der Fluoreszenzlebendauer gibt es jedoch noch weitere Verfahren zur Auswertung von optischen Rohsignalen. Dazu gehört die Auto- und/oder Kreuzkorrelation. Es ist bekannt, daß die Güte einer Korrelationsanalyse linear oder stärker als linear von der Anzahl Photonen abhängt, die pro Partikel und Zeiteinheit registriert werden. Bei einem kommerziellen FCS-Instrument wie dem ConfoCor® werden von einem einzigen Molekül Fluoreszenzfarbstoff Rhodamin 6G bis zu ca. 800 Photonen pro Millisekunde detektiert. Noch mehr Photonen werden detektiert, wenn sich mehrere Luminophore am oder im gleichen Partikel befinden, wie es im Beispiel der Vesikel der Fall ist. In solchen Fällen ist das von Keller et al. beschriebene Verfahren auch mit modernster Elektronik mit vertretbarem Aufwand nicht durchführbar.

Bei einem nicht geglätteten Signal ist es nicht möglich, mehrfach hintereinander die Unterschreitung eines Schwellwertes für den Abstand der Einzelpulse voneinander als Bewertungskriterium zu verwenden, wenn Schwankungen im optischen Rohsignal einzelne Überschreitungen der Abstände verursachen.

Sowohl die in Tellinghuisen et al. als auch in Keller et al. beschriebenen Verfahren versagen, wenn ein partikelklassenspezifischer Signalanteil nicht nur vom Hintergrund, sondern von mindestens einem weiteren partikelspezifischen Signalanteil getrennt werden soll, wie es im eingangs beschriebenen Beispiel des Reaktionsproduktes und der Überschußkomponente der Fall ist. Auch die Überschußkomponente emittiert einen Schauer von Lumineszenzphotonen. Eine schlichte Reduktion des Gesamtsignals um den Streulichtanteil oder die Verwendung einer Schranke bei der Pulsweitendichte lösen die der Erfindung zugrunde liegende Aufgabe nicht.

Die Offenlegungsschrift WO 96/27798 offenbart ein Verfahren zur gleichzeitigen Bestimmung unterschiedlicher Partikelklassen auf der Basis zeitaufgelöster optischer Rohsignale aus mit Fluoreszenzmarkern markierten Molekülen. Diese Bestimmung wird durch die Anwendung statistischer Analysen wie Auto- und krenzkorrelation in der Zeitdomäne erzielt. Die Analyse der optischen Rohdaten bezieht sich jedoch immer auf das gesamte optische Rohsignal.

Der vorliegenden Erfindung liegt die Aufgabe zugrunde, ein Verfahren bereitzustellen, welches die Unterscheidung oder Erfassung von Partikeln in Proben, welche mindestens zwei Partikelklassen enthalten, verbessert.

Die der Erfindung zugrundeliegende Aufgabe wird gelöst durch ein Verfahren mit den Merkmalen des Patentanspruchs 1. Die Patentansprüche 2 bis 18 betreffen bevorzugte Ausführungsformen des erfindungsgemäßen Verfahrens.

Erfindungsgemäß erfolgt die Unterscheidung oder Erfassung von Partikeln in einer Probe, in der auch mehrere Partikelklassen befindlich sein können, durch Identifizierung von Signalabschnitten zeitaufgelöster, optischer Rohsignale aus der Probe auf der Basis von Einzelphotonendetektion (Einzelpulsdetektion), wobei
- die Probe mindestens zwei Partikelklassen enthält,
- die Probe von einer Lichtquelle beleuchtet wird,
- die von der Probe ausgehenden, aus mindestens einem Meßvolumenelement V, wobei V ≤ 10⁻¹² 1, stammenden optischen Rohsignale mittels mindestens einer Detektionseinheit detektiert werden,
- mindestens ein Partikel während seines Aufenthalts im Meßvolumenelement einen Signalanteil erzeugt,
- durch aktiven und/oder passiven Ein- und Wiederaustritt des Partikels in das oder aus dem Meßvolumenelement ein Signalabschnitt der optischen Rohsignale bestimmt wird,
- die optischen Rohsignale in beliebige Abschnitte zerlegt werden,
- für mindestens einen beliebigen Abschnitt mindestens eine auf den optischen Rohsignalen basierende Statistik erstellt wird, und
- die mindestens eine Statistik oder mindestens eine Kombination mehrerer Statistiken nach dem Vorhandensein von Merkmalen, welche für den Signalanteil mindestens einer Partikelklasse charakteristisch sind, bewertet wird.

Im Sinne der Erfindung kann die Bewertung der mindestens einen Statistik oder der mindestens einen Kombination mehrerer Statistiken auch auf Basis solcher Merkmale erfolgen, von denen man annimmt, daß sie für den Signalanteil mindestens einer Partikelklasse charakteristisch sind oder sein könnten.

Erfindungsgemäß wird mindestens eine auf den optischen Rohsignalen basierende Statistik erstellt, wobei diese Vorgehensweise den besonderen Vorteil bietet, daß trotz statistischer Schwankungen im optischen Rohsignal eine verläßliche Bewertung stattfinden kann. Dies steht im Gegensatz zu schlichten Schwellwertentscheidungen.

Es kann wünschenswert sein, daß mindestens ein Hilfssignal gebildet wird, das insbesondere mittels einer Funktion aus dem optischen Rohsignal abgeleitet werden kann und in bevorzugter Weise in zeitlich kausalem Zusammenhang mit dem optischen Rohsignal steht. Dabei kann es bevorzugt sein, daß das Hilfssignal und insbesondere jeweils dessen Wert zu bestimmten, allen, oder fast allen Zeitpunkten statistische Informationen über mindestens einen Abschnitt oder Teilabschnitt des optischen Rohsignals repräsentiert. Es kann wünschenswert sein, daß diese statistische Information einem Grundsignalanteil überlagert ist oder die statistische Information von einer Kombination von Hilfssignalen repräsentiert wird.

Es kann wünschenswert sein, daß mindestens ein Hilfssignal für beliebige Pulse im optischen Rohsignal, insbesondere für jedes detektierte Photon, um einen bestimmten Betrag oder einen Betrag, der u. a. von dem momentanen Wert oder vorausgegangenen Werten des Hilfssignals oder des optischen Rohsignals selbst abhängig sein kann, inkrementiert oder dekrementiert wird.

Es kann weiterhin wünschenswert sein, daß dieses Hilfssignal mit einer Zeitkonstanten, die fest gewählt ist oder u. a. von dem momentanen Wert oder vorausgegangenen Werten des Hilfssignals oder des optischen Rohsignals selbst abhängig sein kann, ab- oder zunimmt. Dabei kann die Ab- oder Zunahme insbesondere linear oder exponentiell sein.

In einer weiteren Ausführungsform des erfindungsgemäßen Verfahrens wird die Amplitude dieses Hilfssignals durch eine Obergrenze und/oder Untergrenze, insbesondere durch Abschneiden, eingeschränkt. Weiterhin ist es möglich, zur Amplitudenbegrenzung eine geeignete Modifikation der Beträge des Inkrements oder Dekrements und/oder der Zeitkonstanten insbesondere in Abhängigkeit des momentanen Wertes oder der vorangegangenen Werte des Hilfssignals vorzunehmen.

Es kann bevorzugt sein, daß bei der Bewertung berücksichtigt wird, ob mindestens ein Hilfssignal innerhalb des Abschnitts oder an der Abschnittsgrenze eine Schwelle erreicht, überoder unterschreitet. Dies kann in einer Ausführungsform das einzige Bewertungskriterium sein, falls das Hilfssignal für mindestens einen Abschnitt oder Teilabschnitt auf den optischen Rohsignalen basierende statistische Informationen enthält.

Es kann wünschenswert sein, daß die Art oder die Kodierung der von den Hilfssignalen repräsentierten statistischen Information entlang der Zeitachse veränderlich ist und insbesondere von den vorausgegangenen oder momentanen Werten dieses oder mindestens eines anderen Hilfssignals oder von mindestens einer Abschnitts- oder Teilabschnittslänge abhängig ist. Es kann wünschenswert sein, daß die Bewertung auf Basis der von mindestens einem Hilfssignal repräsentierten statistischen Information erfolgt.

Es kann bevorzugt sein, die Abschnittsgrenze durch Markierung, insbesondere mittels eines Hilfssignals, oder durch Speicherung der Zeitpunkte oder einer Pulsnummer beziehungsweise Photonen-nummer zu definieren. Insbesondere können somit Abschnitte definiert werden, die einer nachfolgenden Auswertung zugeführt werden. Es kann ferner bevorzugt sein, daß die Abschnittsmarkierung, insbesondere die Markierung eines Abschnittsanfangs, nachträglich gelöscht wird. Dies kann insbesondere bei Abschnitten erfolgen, die bei einer nachfolgenden Auswertung nicht betrachtet werden sollen.

In einer Ausführungsform kann die Statistik in absichtlich oder unabsichtlich verfälschter Form, insbesondere in einer angenäherten Form, erstellt werden.

In einer weiteren Ausführungsform kann die Statistik implizit in ermittelten Daten enthalten sein.

Erfindungsgemäß wird für mindestens einen beliebigen Abschnitt mindestens eine auf den optischen Rohsignalen basierende Statistik erstellt. Im Falle mehrerer beliebiger Abschnitte kann für diese mindestens eine auf den optischen Rohdaten basierende Statistik erstellt werden. So kann beispielsweise für mehrere Abschnitte eine Statistik der Burstdauern erstellt werden.

Mehrere Abschnitte können jedoch erfindungsgemäß auch zusammengefaßt werden, so daß anschließend mindestens eine auf den optischen Rohdaten basierende Statistik hinsichtlich der zusammengefaßten Abschnitte erstellt werden kann.

In einer Ausführungsform kann die Zusammenfassung mehrerer Abschnitte durch Zusammenfassung der Statistikdaten der Einzelabschnitte erfolgen.

In einer weiteren Ausführungsform kann die Zusammenfassung mehrerer Abschnitte durch zeitliche Aneinanderreihung der Abschnitte erfolgen, insbesondere bevor eine Statistik aus diesem zusammengesetzten Signal abgeleitet wird oder bevor die Weitergabe an eine optional nachfolgende Auswertung erfolgt. Dabei kann es wünschenswert sein, daß in dem zusammengesetzten Signal Füllsignalabschnitte eingefügt werden.

Es kann bevorzugt sein, daß die Füllsignalabschnitte eine feste Länge aufweisen oder in ihrer Länge so gewählt werden, daß die einzelnen Abschnitte im zusammengesetzten Signal in der gleichen zeitlichen Beziehung zueinander stehen wie im optischen Rohsignal.

Es kann weiterhin bevorzugt sein, daß als Füllsignal ein Nullsignal, d. h. keine detektierten Photonen, oder ein für das Vorhandensein oder insbesondere das Nichtvorhandensein mindestens einer Partikelklasse im Meßvolumenelement typisches Signal verwendet wird.

Weiterhin kann es bevorzugt sein, als Füllsignal ein Signal zu verwenden, daß aus dem optischen Rohsignal, insbesondere durch Filterung, abgeleitet ist.

Es kann bevorzugt sein, bei der Zerlegung des optischen Rohsignals gleichmäßig lange oder unterschiedlich lange Abschnitte, insbesondere unmittelbar aufeinanderfolgende oder überlappende Abschnitte, zu bilden.

Weiterhin kann es bevorzugt sein, die Zerlegung der optischen Rohsignale in Abschnitte und/oder eine mögliche Auswahl derjenigen Abschnitte, für die eine Statistik erstellt werden soll, auf Basis mindestens einer Funktion des optischen Rohsignals erfolgen zu lassen. Insbesondere kann mindestens eine dieser Funktionen aus dem optischen Rohsignal ein Hilfssignal bilden, welches in bevorzugter Weise in zeitlich kausalem Zusammenhang mit dem optischen Rohsignal steht.

Es kann bevorzugt sein, das optische Rohsignal an denjenigen Zeitpunkten, zu denen mindestens ein Hilfssignal mindestens einen Schwellwert erreicht, überschreitet oder unterschreitet, in Abschnitte zu zerlegen. Eine Zerlegung kann ferner auch in der Nähe dieser Zeitpunkte vorgenommen werden.

Es kann wünschenswert sein, Abschnitte zur Erstellung einer Statistik danach auszuwählen, ob in dem jeweiligen Zeitabschnitt mindestens ein Hilfssignal mindestens einen Schwellwert erreicht, über- oder unterschreitet. Weiterhin kann es wünschenswert sein, eine Auswahl in Abhängigkeit der Länge dieser Abschnitte zu treffen.

Es kann ferner wünschenswert sein, daß die Abschnitte am Anfang und/oder Ende um gewisse Zeiten vergrößert oder verkleinert werden, wobei diese Zeiten insbesondere fest gewählt oder von der Abschnittslänge und/oder Merkmalen des optischen Rohsignals und/oder des Hilfssignals, insbesondere innerhalb des betreffenden Abschnitts und/oder der benachbarten Bereiche, abhängig sein können.

In einer weiteren Ausführungsform kann es bevorzugt sein, daß die Zerlegung der optischen Rohsignale, die Erstellung der Statistik und die Bewertung in einem zyklischen Prozeß erfolgen.

Dabei kann es wünschenswert sein, daß in jedem Zyklus genau ein Abschnitt des optischen Rohsignals betrachtet wird.

Insbesondere in diesem Fall kann es bevorzugt sein, daß wiederholt mindestens ein Abschnitt modifiziert, insbesondere geteilt, mit einem anderen Abschnitt zusammengefaßt, verschoben, verkleinert oder vergrößert wird. Die Verschiebung, Vergrößerung oder Verkleinerung kann insbesondere um eine bestimmte Zeitkonstante, eine infinitesimal kleine Zeiteinheit oder bis zum, bis kurz vor oder hinter dem einer Abschnittsgrenze benachbarten Puls des optischen Rohsignals bzw. benachbarten detektierten Photon erfolgen. Ein derartiges Vorgehen kann auch zur Weitergabe mindestens eines Abschnitts an eine nachfolgende Auswertung bevorzugt sein.

Es kann wünschenswert sein, daß mindestens ein Abschnitt im nachfolgenden Zyklus durch einen anderen, insbesondere zeitlich nachfolgenden, Abschnitt und im besonderen durch einen infinitesimal kleinen Abschnitt, einen Abschnitt bestimmter Länge oder einen eine bestimmte Anzahl Pulse des Rohsignals bzw. detektierte Photonen enthaltenden Abschnitt ersetzt wird.

Bei der Ausführung von Zerlegung, Statistik-Erstellung und Bewertung in einem zyklischen Prozeß kann es wünschenswert sein, daß nur im letzten, in mehreren oder auch in allen Zyklen mindestens ein Abschnitt bestimmt wird, der optional mindestens einer Partikelklasse zugeordnet wird und/oder mindestens einer optional nachfolgenden Auswertung zugeführt wird.

Insbesondere zur Beschleunigung des Verfahrens kann für mindestens einen Abschnitt oder Teilabschnitt mindestens eine Statistik und/oder deren Bewertung von mindestens einer Statistik und/oder deren Bewertung für mindestens einen anderen Abschnitt abgeleitet werden. Dies kann insbesondere bei überlappenden Abschnitten bevorzugt sein. Insbesondere bei einer zyklischen Modifikation von Abschnitten kann es wünschenswert sein, daß mindestens eine Statistik und/oder deren Bewertung aus einem vorausgegangenen Zyklus, insbesondere aus dem unmittelbar vorausgegangenen Zyklus, bei der Ableitung verwendet wird.

In einer bevorzugten Ausführungsform kann für mindestens eine Statistik eines Abschnitts eine Statistik mindestens eines weiteren Abschnitts, insbesondere eines benachbarten Abschnitts oder eines gegenüber dem zu bewertenden Abschnitt vergrößertem oder verkleinertem Abschnitts, zumindest einen Teil der Bewertungsgrundlage darstellen;

In einem derartigen zyklischen Prozeß kann es bevorzugt sein, daß die Bewertung nur in mindestens einem Zyklus nach Merkmalen erfolgt, die für das Vorhandensein mindestens einer Partikelklasse typisch sind. Insbesondere kann es wünschenswert sein, in einem Teil der Zyklen auf die Bewertung und eventuell sogar auf die Erstellung der Statistik zu verzichten.

Es kann bevorzugt sein, daß der Zyklus einmal oder mehrmals durchlaufen wird.

Es kann weiterhin bevorzugt sein, die zyklische Modifikation mindestens eines Abschnitts in einem zyklischen Prozeß abzubrechen oder die Art der Modifikation zu wechseln, wenn mindestens ein Hilfssignal einen Schwellwert erreicht, überoder unterschreitet.

In einer.bevorzugten Ausführungsform werden die optischen Rohsignale nicht physikalisch in Abschnitte zerlegt, sondern die Abschnittseinteilung ist dadurch gegeben, daß mindestens eine auf mindestens einem Abschnitt basierende Statistik erstellt wird.

Es kann bevorzugt sein, daß die Abschnittseinteilung auf Basis des optischen Rohsignals vorgenommen wird, insbesondere daß Abschnitte gebildet werden, die eine feste Anzahl von Pulsen des optischen Rohsignals bzw. detektierte Photonen enthalten. Insbesondere bei einer zyklischen wiederholten Zerlegung in Abschnitte kann es bevorzugt sein, daß initial ein Abschnitt gewählt wird, der genau einen Puls bzw. ein detektiertes Photon enthält.

Es kann weiterhin bevorzugt sein, eine einfache Heuristik zu verwenden, um das optische Rohsignal in Abschnitte zu zerlegen und die Abschnitte zur Erstellung einer Statistik auszuwählen. Insbesondere kann es bevorzugt sein, Abschnitte, die offensichtlich nicht zur Unterscheidung oder Erfassung mindestens einer Partikelklasse geeignet sind, bereits vorzeitig zu verwerfen.

In einer weiteren Ausführungsform kann das erfindungsgemäße Verfahren teilweise oder vollständig, insbesondere unter Verwendung von ASICs oder FPGAs, in Hardware implementiert werden.

In einer weiteren bevorzugten Ausführungsform kann das Verfahren in Echtzeit ausgeführt werden, welches insbesondere für Anwendungen im Bereich des sog. Hochdurchsatzscreenings nach pharmazeutischen Wirkstoffen von besonderem Vorteil ist.

Erfindungsgemäß werden die Photonen einzeln erfaßt (Einzelphotonenzählung oder Einzelpulszählung). Die Registrierung der Zählerpulse kann auf unterschiedliche Weise erfolgen. So kann z. B. jedem registrierten Einzelpuls der Zeitpunkt der Registrierung durch den Detektor zugeordnet werden oder aber die Anzahl der registrierten Einzelpulse in einem vorgegebenen Zeitintervall ermittelt werden. Es kann bevorzugt sein, die Einzelpulse auf mehreren Detektoren zu registrieren und/oder parallel dazu weitere Signale zu registrieren, die in physikalischem Bezug zu den registrierten Einzelpulsen stehen und so zusätzliche Informationen liefern.

Die Photonen werden von Partikeln emittiert, die in das Meßvolumenelement V von V ≤ 10⁻¹² 1 eintreten und wieder austreten. Ein besonderer Vorteil des kleinen Meßvolumenelementes ist darin zu sehen, daß sich selbst bei Konzentrationen im nanomolaren Bereich nur wenige Partikel im Meßvolumenelement befinden. Besonders deutlich wird dieser Effekt bei größeren Partikeln wie z. B. zellulären Membranfragmenten oder -vesikeln, die maximal im pikomolaren Konzentrationsbereich herstellbar sind. Um bei diesen Konzentrationen einen homogenen Assay z. B. der Bindung von farbstoffmarkierten Liganden an Rezeptoren in Membranfragmenten durchführen zu können, muß die Ligandenkonzentration in bedeutendem Überschuß gewählt werden. Dies bedeutet, daß sich den größten Teil der Meßzeit im Meßvolumenelement nur freie Liganden befinden und nur selten das Reaktionsprodukt in Form von ligandenbindenden Membranvesikeln. Während des Aufenthalts des Membranvesikels in einem kleinen Meßvolumenelement dominiert sein Signalanteil bei geeignet gewählten Konzentrationen jedoch das registrierte optische Rohsignal, so daß der Signalanteil der Partikelklasse der freien Liganden und des freien Farbstoffes demgegenüber in erster Näherung vernachlässigt werden kann. In zweiter Näherung kann es bevorzugt sein, ihn bei der Bewertung oder Auswertung des Signalabschnittes zu berücksichtigen. In einem größeren Meßvolumenelement befinden sich dagegen immer sowohl freie Liganden und freier Farbstoff als auch Membranfragmente. In Folge der Mittelung über mehrere Partikel sind in diesem Fall beide Signalanteile in allen beliebigen Abschnitten fast gleich, so daß eine Identifikation der verschiedenen Signalanteile nach Partikelklassen nun nicht mehr möglich wäre.

Das kleine Meßvolumenelement allein reicht zur Identifikation der Signalabschnitte noch nicht aus, wenn sich mehr als eine Partikelklasse in der Probe befindet und die Identifikation nur anhand von den in der Literatur beschriebenen Schwellwertentscheidungen stattfindet. Hintergrundsignale, wie etwa das Rauschen des Detektors oder das Ramansignal des Lösungsmittels, welches selbst in einem kleinen Meßvolumenelement immer in hoher Konzentration vorhanden ist, geben ein gleichförmiges Signal, von dem sich jeder burst deutlich abhebt und über einfache Schwellwertentscheidungen identifizieren läßt. Sind mehr als zwei Partikelklassen im Meßvolumenelement vorhanden, so werden beide unterschiedslos als burst erkannt. Erst das erfindungsgemäße Erstellen einer auf den optischen Rohsignalen basierenden Statistik für mindestens einen Abschnitt oder Teilabschnitt ermöglicht die Unterscheidung oder Erfassung der Partikel nach mehr als einer Partikelklasse.

In einer Ausführungsform des erfindungsgemäßen Verfahrens sind die Partikel mindestens einer Partikelklasse lumineszent und/ oder durch mindestens einen Luminophor markiert. Die Partikel einer Partikelklasse können insbesondere Moleküle, Makromoleküle, Viren, Bakterien, Zellen, Beads, Vesikel, Partikelfluide, Molekülaggregate oder -komplexe sein. Eine Partikelklasse kann mehrere Sorten Partikel enthalten. Beispielsweise sind bei der Messung der Bindung eines markierten Liganden an Rezeptoren auf Vesikeln häufig noch freie Luminophore in der Probe zugegen. In diesem Fall ist es wünschenswert, die Signalabschnitte, in denen ein Signalanteil vorkommt, der von rezeptorgebundenen Liganden stammt, vom Rest des optischen Rohsignals zu unterscheiden. Entsprechend würden Vesikel zu einer Partikelklasse gezählt und markierte freie Liganden sowie freie Luminophore zu einer weiteren. Es ist hierbei hervorzuheben, daß eine Partikelklasse durchaus eine ganze Verteilung von z. B. Partikelgrößen enthalten kann, wie dies bei Membranfragmenten oder Vesikeln häufig der Fall ist.Insbesondere gehören verschiedene Partikelsorten verschiedenen Partikelklassen an, wenn diese verschiedenen Partikelsorten bei der Identifikation der Signalabschnitte und/oder bei der optional nachfolgenden Auswertung unterschieden werden.

Es befinden sich mindestens zwei Partikelklassen in der Probe, deren Partikel insbesondere zu unvorhersehbaren Zeitpunkten in das Meßvolumenelement eintreten und zu unvorhersehbaren oder vorhersehbaren Zeitpunkten wieder austreten.

Erfindungsgemäß wird für mindestens einen beliebigen Abschnitt eine auf den optischen Rohsignalen basierende Statistik erstellt. Hierbei kann es sich in einer bevorzugten Ausführungsform um die Erstellung einer Statistik der Zahl der Einzelpulse und/oder der zeitlichen Abstände der Einzelpulse, insbesondere der charakteristischen Triplettzeitverteilung und/oder Tripletthäufigkeit der Luminophore, und/oder des elastisch und/oder inelastisch gestreuten Lichtes handeln.

Beispielsweise hängt die Häufigkeit und die Verweildauer eines Luminophores im Triplettzustand von seiner chemischen Umgebung ab. Häufig ändert sie sich bei der Bindung des markierten Partikels an ein weiteres Partikel und eignet sich so zu deren Identifizierung.

Die Streuung des Anregungslichtes ist ein weiteres Beispiel für eine Identifikation nach Partikelklassen. Freie Liganden erzeugen kaum Streulicht; größere Partikel wie Membranfragmente oder beads erzeugen dagegen soviel, daß ihr Signal detektierbar ist. Die Intensität des gestreuten Lichts kann sogar zur Normierung von weiteren, z. B. Lumineszenzsignalen auf die Größe der Partikel verwendet werden. Ein solches Vorgehen trägt erheblich zu einer genaueren Bestimmung der Bindungskonstante bei. Im Beispiel der Membranfragmente und der markierten Liganden läßt sich bei geeignet gewählter Konzentration der Liganden aus der Statistik der Größe der Membranfragmente und der registrierten Einzelpulse sogar auf die Dichte der Rezeptoren pro Membranfläche schließen. Diese Größe ist von großer praktischer Bedeutung in der Forschung und anders einer experimentellen Bestimmung kaum zugänglich. Statt des Streulichtes kann auch die Lumineszenz eines zweiten Luminophores verwendet werden, der sich in die Membran einlagert, ohne mit dem Liganden in Wechselwirkung zu treten.

Es kann bevorzugt sein, die Art der verwendeten Statistik und/oder eine Kombination von mehr als einer Statistik je nach Art der zu identifizierenden Partikelklasse und ihrer Unterscheidungsmerkmale zu wählen.

Das folgende Beispiel mag ein solches Vorgehen erläutern. Die Partikelklassen eines homogenen Assays mögen wie in dem eingangs genannten Beispiel wiederum rezeptortragende Vesikel einerseits und freie, lumineszente oder lumineszent markierte Liganden sein, die gemäß einer Gleichgewichtskonstante zu einem bestimmten Prozentsatz an die Rezeptoren binden. Freie Liganden, die sich durch das Meßvolumenelement bewegen, erzeugen im Mittel relativ kurze bursts hoher Intensität, Vesikel wegen ihrer geringen Diffusionsgeschwindigkeit im Mittel relativ lange. Eine einfache wie in der Literatur beschriebene Schwellwertentscheidung, welche bewertet, ob mehrfach hintereinander Einzelpulse innerhalb einer vorgegebenen kurzen Zeit registriert werden, würde zur Identifizierung nicht ausreichen. Bei einer zu niedrig gesetzten Schranke zählt sie beide Partikelklassen zusammen, bei einer höheren Schranke wird das Schwellwertkriterium dagegen fast nie erfüllt, weil sich wegen der statistischen Schwankungen auch bei den Vesikeln immer wieder Pausen ergeben.

Erst die Erstellung einer Statistik, z. B. der Größe I(Δt)I(Δt+t₁)I(Δt+t₂)I(Δt+t₃) mit der Anzahl I der jeweils während der Abschnitte Δt, Δt+t₁, Δt+t₂ und Δt+t₃ gezählten Einzelpulse liefert bei geeignet gewählten Konstanten tᵢ mit i=1,2,3 ein statistisch sicheres Merkmal, anhand dessen die Partikelklassen voneinander unterschieden werden können.

Es kann bevorzugt sein, die Probe mit polarisiertem Anregungslicht zu beleuchten und eine Statistik der Polarisation zu erstellen.

Zum Beispiel kann bei Verwendung zweier Detektoren mit zueinander gekreuzten Polarisationsfiltern die Statistik des Verhältnisses der gezählten Einzelpulse zueinander erstellt werden.

In einer weiteren Ausführungsform ist es möglich, die Probe mit gepulstem oder zeitlich moduliertem Anregungslicht zu beleuchten und eine Statistik der zeitlichen Abstände der Einzelpulse von den Anregungspulsen oder von der Phase des Anregungslichtes zu erstellen.

Auch ist eine Parallelisierung der Photonenregistrierung denkbar, z. B. Lumineszenz bei verschiedenen Wellenlängen oder die zusätzliche Registrierung von gestreutem oder Ramanlicht oder eine beliebige Kombination dieser Parameter.

Es kann in einer weiteren Ausführungsform wünschenswert sein, Abschnitte und/oder Teile dieser Abschnitte der optischen Rohsignale und/oder der bereits erstellten, mindestens einen Statistik mindestens einer Partikelklasse zuzuordnen, sowie ferner diese zu sortieren und mindestens einer Auswertung zur Bestimmung von chemischen und/oder physikalischen Parametern, die spezifisch für die mindestens eine Partikelklasse sind, zuzuführen. Die Auswertung kann in Form einer zeitlichen Mittelung und/oder einer Bestimmung höherer statistischer Momente und/oder der Bestimmung der Autound/oder Kreuzkorrelation und/oder Fouriertransformation und/oder Analyse einer Verteilungsfunktion der optischen Rohsignale der (Teil-)Abschnitte und/oder der Länge der (Teil-)Abschnitte, und/oder der Lumineszenzdepolarisation, und/oder der Lumineszenzlebensdauer erfolgen.

Es kann bevorzugt sein, die Länge der bursts der mindestens einen Partikelklasse und ihrer korrespondierenden Pulsstatistik nach der Sortierung einer weiteren statistischen Auswertung zu unterwerfen.

In einer bevorzugten Ausführungsform wird die Lumineszenz zweier spektral verschiedener Luminophore mit zwei Detektoren parallel gemessen, beispielsweise in einer Anordnung wie der in WO 94/16313 beschriebenen. Das erfindungsgemäße Verfahren in Kombination mit der Kreuzkorrelation als weiterem Auswertungsschritt nach der Sortierung des optischen Rohsignals profitiert besonders von der Sortierung, da sich nach der Anwendung boolescher Funktionen wie beispielsweise "und" ("AND") und/oder "negiertes und" ("NAND") Korrelationen besser bestimmen lassen. Vor allem kann eine Korrelation über die Präsenz ausschließlich einer Farbe im Meßvolumenelement bestimmt werden, was kein anderes bekanntes Verfahren ermöglicht.

Insbesondere ist es möglich, Kreuzkorrelationen und/oder andere bedingte Statistiken auch mit nur einem Detektor zu bestimmen. Bei geeigneten optischen Rohsignalen erlaubt die Statistik zu entscheiden, ob der Abschnitt nur einer oder mehreren Partikelklassen zugeordnet werden soll. Werden zum Beispiel genau zwei Partikelklassen A und B unterschieden, so kann die Sortierung nach den drei Fällen (nur A), (nur B) sowie (A und B) erfolgen. Es kann sogar bevorzugt sein, in diesem Beispiel noch eine vierte Klasse (weder A noch B) zu unterscheiden.

In einer weiteren Ausführungsform des erfindungsgemäßen Verfahrens sind die Lichtquelle und die Detektionseinheit konfokal bezüglich des Meßvolumenelementes angeordnet.

In einigen Anwendungsfällen mag es bevorzugt sein, die lumineszenten oder mit einem Luminophor markierten Partikel durch 2-Photonenanregung zur Emission zu stimulieren.

Eine weitere Ausführungsform ist dadurch gekennzeichnet, daß das Licht zwischen Lichtquelle und Probe und/oder Probe und Detektionseinheit eine für die entsprechende Wellenlänge des Lichtes und/oder des von der Probe ausgehenden Lichts durchlässige Einrichtung passiert, wobei diese Einrichtung in unmittelbarer Nähe der Probe angebracht ist und mindestens einen Bereich aufweist, dessen größte Ausdehnung in mindestens einer Raumrichtung kleiner ist als die Wellenlänge des Lichtes und/oder des von der Probe ausgehenden Lichts. Weitere Einzelheiten hierzu sind in der WO 96/13744 beschrieben.

In einer weiteren Ausführungsform wird das erfindungsgemäße Verfahren mit dem in der europäischen Patentanmeldung 96 116 373.0 beschriebenen Verfahren kombiniert. Auf den Offenbarungsgehalt dieser Patentanmeldung wird hiermit ausdrücklich Bezug genommen.

Bei stark eingeschränkter Diffusion der Partikel mindestens einer Partikelklasse kann es bevorzugt sein, eine erzwungene Bewegung der Probe und des darin erhaltenen Meßvolumenelementes (z. B. durch Vibration oder Fluß) und/oder eine kontinuierliche oder diskontinuierliche Veränderung der Ortskoordinaten des Meßvolumenelementes innerhalb der Probe zu erzeugen. Dies geschieht vorzugsweise durch eine Veränderung des Fokus und/oder durch eine Veränderung der Position des ausgeleuchteten Volumenelementes. Weitere Einzelheiten hierzu sind in der WO 94/16313 beschrieben.

In einer bevorzugten Ausführungsform wird die Veränderung der Ortskoordinaten parallel zu den Einzelpulsen registriert und/ oder aus Geräteparametern berechnet. Sie kann bei der Erstellung der mindestens einen auf optischen Rohsignalen basierenden Statistik des mindestens einen Abschnitts und/oder Teilabschnitts verwendet werden. Beispielsweise kann bei einer periodischen oder quasiperiodischen Bewegung, bei der das Meßvolumen sich mindestens zweimal während der Messung am gleichen Ort der Probe befindet, der mindestens eine Abschnitt und/oder Teilabschnitt für die gleiche Statistik verwendet werden.

In einer weiteren Ausführungsform variieren die molaren Konzentrationen der zu unterscheidenden Partikel um einen Faktor größer 10.

Es kann weiterhin bevorzugt sein, die chemischen und/oder physikalischen Eigenschaften der Oberfläche von mindestens einem Partikel über Lumineszenzeigenschaften zu bestimmen. Insbesondere kann die Dichte der Rezeptoren pro Membranfläche bestimmt werden.

Die nachfolgenden Figuren verdeutlichen das erfindungsgemäße Verfahren.

Die in den Figuren dargestellten Verhältnisse beziehen sich auf Messungen an mit Rhodamin-6G markiertem EGF (epidermal growth factor; 10 nmolar in wäßriger Lösung) und EGF-Rezeptor-tragenden Vesikeln (7,5 nmolar).

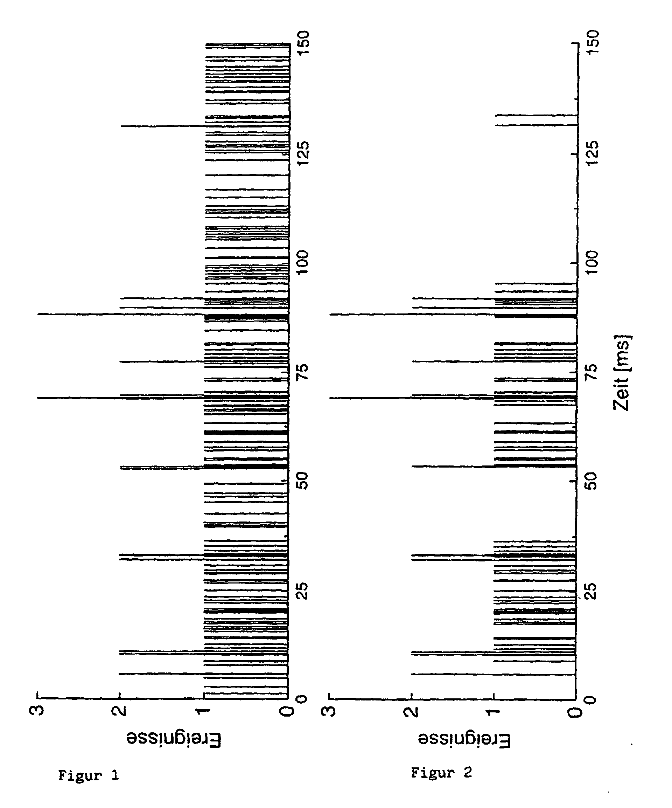

Figur 1 zeigt das optische Rohsignal. Detektiert wurden Signale in Abhängigkeit von der Zeit.

Figur 2 zeigt eine Auswahl derjenigen Abschnitte, für die eine Statistik erstellt werden soll.

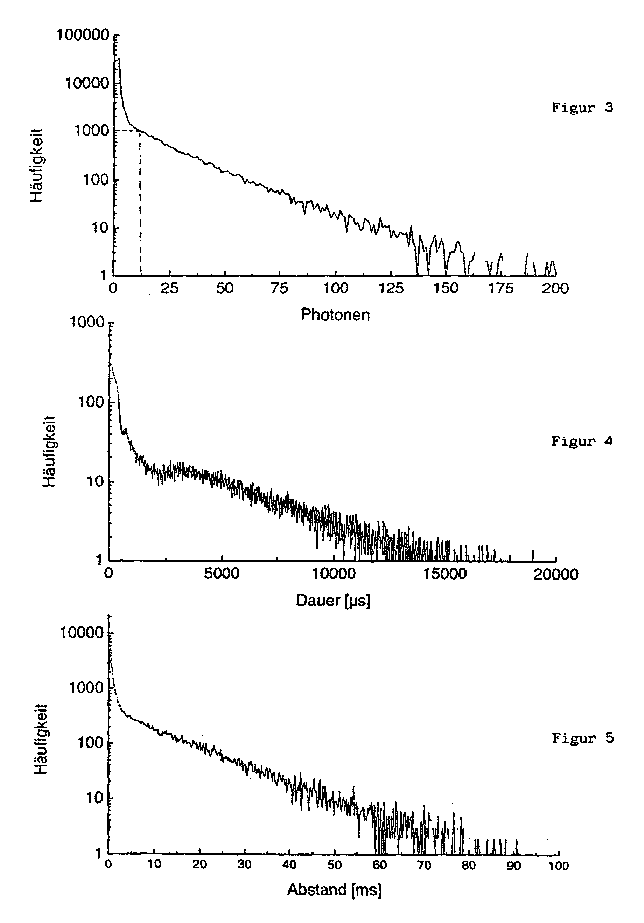

Figur 3 zeigt eine Häufigkeitsverteilung über die in den Abschnitten aufgetretene Photonenanzahl. Eine Abschätzung aus dem Diagramm ergibt, daß in etwa 12 Photonen ca. 1.000 mal auftraten.

Figur 4 zeigt eine Häufigkeitsverteilung über die zeitliche Länge der Abschnitte (siehe Figur 2).

Figur 5 zeigt eine Häufigkeitsverteilung über die zeitlichen Abstände zwischen den Abschnitten (siehe Figur 2).

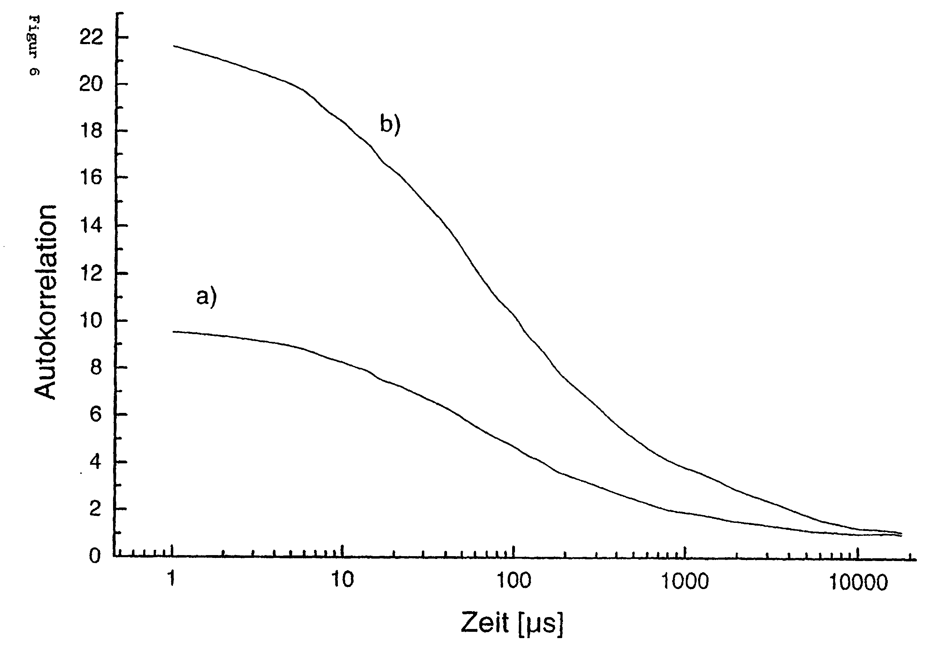

Figur 6 zeigt einen Vergleich der Autokorrelationsfunktionen des optischen Rohsignals (a) und des erfindungsgemäß verarbeiteten Signals (b).

## Patentansprüche

1. Verfahren zur Unterscheidung oder Erfassung von Partikeln in einer Probe, in der auch mehrere Partikelklassen befindlich sein können, durch Identifizierung von Signalabschnitten zeitaufgelöster, optischer Rohsignale aus der Probe auf der Basis von Einzelphotonendetektion (Einzelpulsdetektion), wobei
- die Probe mindestens zwei Partikelklassen enthält,
- die Probe von einer Lichtquelle beleuchtet wird,
- die von der Probe ausgehenden, aus mindestens einem Meßvolumenelement V, wobei V ≤ 10⁻¹² 1, stammenden optischen Rohsignale mittels mindestens einer Detektionseinheit detektiert werden,
- mindestens ein Partikel während seines Aufenthalts im Meßvolumenelement einen Signalanteil erzeugt,
- durch aktiven und/oder passiven Ein- und Wiederaustritt des Partikels in das oder aus dem Meßvolumenelement ein Signalabschnitt der optischen Rohsignale bestimmt wird,
- die optischen Rohsignale in beliebige Abschnitte zerlegt werden,
- für mindestens einen beliebigen Abschnitt mindestens eine auf den optischen Rohsignalen basierende Statistik erstellt wird, und
- die mindestens eine Statistik oder mindestens eine Kombination mehrerer Statistiken nach dem Vorhandensein von Merkmalen, welche für den Signalanteil mindestens einer Partikelklasse charakteristisch sind, bewertet wird.

2. Verfahren nach Anspruch 1, **dadurch gekennzeichnet, daß** Partikel mindestens einer Partikelklasse lumineszent sind und/oder durch mindestens einen Luminophor markiert sind.

3. Verfahren nach Anspruch 1 und/oder 2, **dadurch gekennzeichnet, daß** die Partikel einer Partikelklasse Moleküle, Makromoleküle, Viren, Bakterien, Zellen, Beads, Vesikel, Partikelfluide, Molekülaggregate oder -komplexe sind.

4. Verfahren nach mindestens einem der Ansprüche 1 bis 3, **dadurch gekennzeichnet, daß** eine Statistik der Zahl der Einzelpulse und/oder der zeitlichen Abstände der Einzelpulse, insbesondere der charakteristischen Triplettzeitverteilung und/oder Tripletthäufigkeit der Luminophore, und/oder des elastisch und/oder inelastisch gestreuten Lichtes erstellt wird.

5. Verfahren nach mindestens einem der Ansprüche 1 bis 4, **dadurch gekennzeichnet, daß** die Probe mit polarisiertem Anregungslicht beleuchtet wird und daß eine Statistik der Polarisation erstellt wird.

6. Verfahren nach mindestens einem der Ansprüche 1 bis 5, **dadurch gekennzeichnet, daß** die Probe mit gepulstem oder zeitlich moduliertem Anregungslicht beleuchtet wird und daß eine Statistik der zeitlichen Abstände der Einzelpulse von den Anregungspulsen oder von der Phase des Anregungslichtes erstellt wird.

7. Verfahren nach mindestens einem der Ansprüche 1 bis 6, **dadurch gekennzeichnet, daß** Abschnitte und/oder Teile dieser Abschnitte und/oder der mindestens einen erstellten Statistik mindestens einer Partikelklasse zugeordnet werden.

8. Verfahren nach mindestens einem der Ansprüche 1 bis 7, **dadurch gekennzeichnet, daß** die Abschnitte und/oder Teile dieser Abschnitte und/oder der mindestens einen erstellten Statistik nach ihrer Zuordnung zu mindestens einer Partikelklasse sortiert und mindestens einer Auswertung zur Bestimmung von chemischen und/oder physikalischen Parametern, die spezifisch für die mindestens eine Partikelklasse sind, zugeführt werden.

9. Verfahren nach mindestens einem der Ansprüche 1 bis 8, **dadurch gekennzeichnet, daß** die Auswertung in Form einer zeitlichen Mittelung und/oder einer Bestimmung höherer statistischer Momente und/oder der Bestimmung der Auto- und/oder Kreuzkorrelation und/oder Fouriertransformation und/oder Analyse einer Verteilungsfunktion der optischen Rohsignale der (Teil)Abschnitte und/oder der Länge der (Teil-)Abschnitte, und/oder der Lumineszenzdepolarisation, und/oder der Lumineszenzlebensdauer erfolgt.

10. Verfahren nach mindestens einem der Ansprüche 1 - 9, **dadurch gekennzeichnet, daß** nach einer Sortierung des optischen Rohsignals und/oder der mindestens einen erstellten Statistik das mindestens eine sortierte Signal mit mindestens einer booleschen Funktion bewertet wird und gegebenenfalls anschließend einer weiteren Auswertung unterworfen werden.

11. Verfahren nach mindestens einem der Ansprüche 1 bis 10, **dadurch gekennzeichnet, daß** eine konfokale Anordnung von.Lichtquelle und Detektionseinheit bezüglich des Meßvolumen-elementes verwendet wird.

12. Verfahren nach mindestens einem der Ansprüche 1 bis 11, **dadurch gekennzeichnet, daß** die lumineszenten oder mit einem Luminophor markierten Partikel durch 2-Photonenanregung zur Emission stimuliert werden.

13. Verfahren nach mindestens einem der Ansprüche 1 bis 12, **dadurch gekennzeichnet, daß** das Licht zwischen Lichtquelle und Probe und/oder Probe und Detektionseinheit eine für die entsprechende Wellenlänge des Lichtes und/oder des von der Probe ausgehenden Lichts durchlässige Einrichtung passiert, wobei diese Einrichtung in unmittelbarer Nähe der Probe angebracht ist und mindestens einen Bereich aufweist, dessen größte Ausdehnung in mindestens einer Raumrichtung kleiner ist als die Wellenlänge des Lichtes und/oder des von der Probe ausgehenden Lichts.

14. Verfahren nach mindestens einem der Ansprüche 1 bis 13, **dadurch gekennzeichnet, daß** die Ortskoordinaten des Meßvolumenelementes relativ zu den Ortskoordinaten der Probe während der Messung verändert werden, um stationäre oder sehr langsam diffundierende lumineszierende oder lumineszent markierte Partikel zu messen.

15. Verfahren nach mindestens einem der Ansprüche 1 bis 14, **dadurch gekennzeichnet, daß** die Veränderung der Ortskoordinaten parallel zu den optischen Rohsignalen registriert wird und bei der Erstellung der mindestens einen Statistik berücksichtigt wird, insbesondere bei periodischen oder quasiperiodischen Veränderungen der Ortskoordinaten.

16. Verfahren nach mindestens einem der Ansprüche 1 bis 15. **dadurch gekennzeichnet, daß** die molaren Konzentrationen der zu unterscheidenden Partikel um einen Faktor größer 10 variieren.

17. Verfahren nach mindestens einem der Ansprüche 1 bis 16, **dadurch gekennzeichnet, daß** die chemischen und/oder physikalischen Eigenschaften der Oberfläche von mindestens einem Partikel über Lumineszenzeigenschaften bestimmt werden.

18. Verfahren nach mindestens einem der Ansprüche 1 bis 17, **dadurch gekennzeichnet, daß** die Dichte von Rezeptoren pro Membranfläche bestimmt wird.

## Claims

1. A method for differentiating or detecting particles in a sample in which several classes of particles may be present by identifying signal segments of time-resolved, optical raw signals from the sample on the basis of single photon detection (single pulse detection), wherein
- the sample contains at least two classes of particles;
- the sample is illuminated by a light source;
- the optical raw signals emitted by the sample, which are derived from at least one measuring volume element V, V ≤ 10⁻¹² I, are detected with at least one detector unit;
- at least one particle generates a signal fraction during its residence in the measuring volume element;
- a signal segment of the optical raw signals is determined by the particle's actively and/or passively entering and then leaving again the measuring volume element;
- the optical raw signals are segmented into arbitrary segments;
- at least one set of statistical data based on the optical raw signals is established for at least one arbitrarily chosen segment; and
- said at least one set of statistical data or at least one combination of several sets of statistical data is evaluated for the presence of features characteristic of the signal fraction from at least one class of particles.

2. The method according to claim 1, **characterized in that** particles of at least one class of particles are luminescent and/or labeled with at least one luminophor.

3. The method according to claim 1 and/or 2, **characterized in that** particles of a class of particles are molecules, macromolecules, viruses, bacteria, cells, beads, vesicles, particle fluids, molecular aggregates or complexes.

4. The method according to at least one of claims 1 to 3, **characterized in that** a set of statistical data is established for the number of individual pulses and/or the time intervals between the individual pulses, especially the characteristic triplet time distribution and/or triplet frequency of the luminophors and/or the elastically and/or inelastically scattered light.

5. The method according to at least one of claims 1 to 4, **characterized in that** the sample is illuminated with polarized excitation light and a set of statistical data for polarization is established.

6. The method according to at least one of claims 1 to 5, **characterized in that** the sample is illuminated with pulsed or temporally modulated excitation light and that a set of statistical data is established for the time intervals to the individual pulses from the excitation pulses or from the phase of the excitation light.

7. The method according to at least one of claims 1 to 6, **characterized in that** segments and/or portions of said segments and/or of the at least one established set of statistical data are assigned to at least one class of particles.

8. The method according to at least one of claims 1 to 7, **characterized in that** said segments and/or portions of said segments and/or of the at least one established set of statistical data, after having been assigned to at least one class of particles, are classified and subjected to at least one evaluation for determining chemical and/or physical parameters which are specifically of said at least one class of particles.

9. The method according to at least one of claims 1 to 8, **characterized in that** said evaluation is done in the form of time-averaging, and/or the determination of higher statistical moments, and/or the determination of the auto- and/or cross-correlation, and/or Fourier transformation, and/or analysis of a distribution function of the optical raw signals of the (sub)segments and/or the length of the (sub)segments and/or luminescence depolarization and/or luminescence lifetime.

10. The method according to at least one of claims 1 to 9, **characterized in that**, after classifying the optical raw signal and/or the at least one established set of statistical data, said at least one classified signal is evaluated by at least one Boolean function and then optionally subjected to further evaluation.

11. The method according to at least one of claims 1 to 10, **characterized in that** a confocal arrangement of the light source and the detector unit with respect to the measuring volume element is used.

12. The method according to at least one of claims 1 to 11, **characterized in that** said luminescent or luminophor-labeled particles are stimulated to emission by two-photon excitation.

13. The method according to at least one of claims 1 to 12, **characterized in that** the light passes a means which is transparent to the corresponding wavelength of the light and/or the light emitted by the sample, which means is provided between the light source and the sample and/or between the sample and the detector unit, in close proximity of the sample, and has at least one region the largest dimension of which in at least one direction of space is smaller than the wavelength of the light and/or the light emitted by the sample.

14. The method according to at least one of claims 1 to 13, **characterized in that** the space coordinates of the measuring volume element are changed relatively to the space coordinates of the sample during measurement to measure stationary or very slowly diffusing luminescent or luminescence-labeled particles.

15. The method according to at least one of claims 1 to 14, **characterized in that** said change of the space coordinates is recorded in parallel with the optical raw signals and considered in establishing said at least one set of statistical data, especially for periodic or quasi-periodic changes of the space coordinates.

16. The method according to at least one of claims 1 to 15, **characterized in that** the molar concentrations of the particles to be differentiated vary by a factor of more than 10.

17. The method according to at least one of claims 1 to 16, **characterized in that** the chemical and/or physical properties of the surface of at least one particle are determined using luminescence properties.

18. The method according to at least one of claims 1 to 17, **characterized in that** the density of receptors per membrane area is determined.

## Revendications

1. Procédé de différentiation ou détection de particules dans un échantillon dans lequel plusieurs classes de particules peuvent être présentes, par l'identification de segments de signaux provenant de signaux bruts optiques résolus en temps obtenus de l'échantillon à la base de détection de photons singuliers (détection single-pulse), dans lequel:
- l'échantillon contient au moins deux classes de particules;
- l'échantillon est illuminé par une source de lumière;
- lesdits signaux bruts optiques émis par l'échantillon, qui sont dérivés d'au moins un élément de volume de mesurage V, V ≤ 10⁻¹² |, sont détectés avec au moins une unité détectrice;
- au moins une particule génère une fraction de signal pendant son séjour dans l'élément de volume de mesurage;
- un segment de signal des signaux bruts optiques est déterminé au moyen du fait que la particule entre dans l'élément de volume de mesurage et en ressort activement ou passivement;
- les signaux bruts optiques sont segmentés en segments arbitraires;
- au moins un ensemble de dates statistiques à la base des signaux bruts optiques est. établi pour au moins un segment choisi arbitrairement; et
- ledit au moins un ensemble de dates statistiques ou au moins une combinaison de plusieurs ensembles de dates statistiques est évalué pour la présence de caractéristiques de la fraction de signal provenant d'au moins une classe de particules.

2. Procédé selon la revendication 1, **caractérisé en ce que** particules d'au moins une classe de particules sont luminescentes et/ou marquées avec au moins un luminophore.

3. Procédé selon l'une des revendications 1 et/ou 2, **caractérisé en ce que** particules d'une classe de particules sont des molécules, des macromolécules, des virus, des bactéries, des cellules, des perles, des vésicules, des fluides de particules, des agrégats moléculaires ou des complexes.

4. Procédé selon au moins une des revendications 1 à 3, **caractérisé en ce qu'**un ensemble de dates statistiques est établi pour le nombre d'impulsions individuelles et/ou les intervalles de temps entre les impulsions individuelles, notamment la distribution en temps de triplets caractéristique et/ou la fréquence de triplets des luminophores et/ou la lumière diffusée élastiquement ou inélastiquement.

5. Procédé selon au moins une des revendications 1 à 4, **caractérisé en ce que** l'échantillon est illuminé avec de la lumière d'excitation polarisée, et un ensemble de dates statistiques est établi pour la polarisation.

6. Procédé selon au moins une des revendications 1 à 5, **caractérisé en ce que** l'échantillon est illuminé avec de la lumière d'excitation pulsée ou modulée en temps, et qu'un ensemble de dates statistiques est établi pour les intervalles de temps des impulsions d'excitation ou de la phase de la lumière d'excitation aux impulsions individuelles.

7. Procédé selon au moins une des revendications 1 à 6, **caractérisé en ce que** des segments et/ou des parties desdits segments et/ou dudit au moins un ensemble de dates statistiques établi sont attribués à au moins une classe de particules.

8. Procédé selon au moins une des revendications 1 à 7, **caractérisé en ce que** lesdits segments et/ou parties desdits segments et/ou dudit au moins un ensemble de dates statistiques établi, après être attribués à au moins une classe de particules, sont classifiés et soumis à au moins une évaluation pour déterminer des paramètres chimiques et/ou physiques qui sont spécifiques à ladite au moins une classe de particules.

9. Procédé selon au moins une des revendications 1 à 8, **caractérisé en ce que** ladite évaluation est effectuée par la moyenne en temps, et/ou par la détermination de moments statistiques supérieurs, et/ou par la détermination d'auto-corrélation ou de corrélation croisée, et/ou par transformation Fourier, et ou par analyse d'une fonction de distribution des signaux bruts optiques des (sous)segments et/ou de la longueur des (sous)segments et/ou de la dépolarisation de luminescence et/ou de la durée de vie de luminescence.

10. Procédé selon au moins une des revendications 1 à 9, **caractérisé en ce que**, après la classification les signaux bruts optiques et/ou ledit au moins un ensemble de dates statistiques établi, ledit au moins un signal classifié est évalué par au moins une fonction de Boole et puis éventuellement soumis à une autre évaluation.

11. Procédé selon au moins une des revendications 1 à 10, **caractérisé en ce qu'**un arrangement confocal de la source de lumière et de l'unité détectrice par rapport au volume de mesurage est utilisé.

12. Procédé selon au moins une des revendications 1 à 11, **caractérisé en ce** lesdites particules luminescentes ou marquées avec un luminophore sont stimulées pour émission par une excitation à deux photons.

13. Procédé selon au moins une des revendications 1 à 12, **caractérisé en ce** la lumière passe un moyen qui est transparent à la longueur d'onde correspondante de la lumière et/ou de la lumière émise par l'échantillon, ledit moyen étant placé entre la source de lumière et l'échantillon et/ou entre l'échantillon et l'unité détectrice, tout proche de l'échantillon, et ayant au moins une région dont la dimension la plus grande dans au moins une direction spatiale est plus petite que la longueur d'onde de la lumière et/ou de la lumière émise par l'échantillon.

14. Procédé selon au moins une des revendications 1 à 13, **caractérisé en ce** les coordonnées spatiales de l'élément de volume de mesurage sont changées relativement aux coordonnées spatiales de l'échantillon pendant le mesurage pour mesurer des particules luminescentes ou marquées avec un luminophore qui sont stationnaires ou qui diffusent lentement.

15. Procédé selon au moins une des revendications 1 à 14, **caractérisé en ce** ledit changement des coordonnées spatiales est enregistré parallèlement avec les signaux bruts optiques et considéré quand ledit au moins un ensemble de dates statistiques est établi, notamment pour changements de coordonnées spatiales périodiques ou quasi-périodiques.

16. Procédé selon au moins une des revendications 1 à 15, **caractérisé en ce** les concentrations molaires des particules à différencier varient par un facteur de plus de 10.

17. Procédé selon au moins une des revendications 1 à 16, **caractérisé en ce** les propriétés chimiques et/ou physiques de la surface d'au moins une particule sont déterminées au moyen de propriétés de luminescence.

18. Procédé selon au moins une des revendications 1 à 17, **caractérisé en ce** la densité de récepteurs par superficie de membrane est déterminée.
